# EUROPEAN PATENT SPECIFICATION

(11) **EP 4 213 818 B1**
(45) Date of publication and mention of the grant of the patent: **11.03.2026**
(21) Application number: 21773782.4
(22) Date of filing: 09.09.2021
(51) Int. Cl.: A61K 31/122, A61K 31/4178, A61K 9/00, A61P 33/00

(54) **AN OPTIMISED VETERINARY INJECTABLE FORMULATION**
OPTIMIERTE VETERINÄRMEDIZINISCHE INJIZIERBARE FORMULIERUNG
FORMULATION VÉTÉRINAIRE INJECTABLE OPTIMISÉE

(30) Priority: 14.09.2020 EP 20196085
(43) Date of publication of application: 26.07.2023
(73) Proprietor: Bimeda AMEA Limited, Dublin D18 K8Y4 (IE)
(72) Inventor: YANG, Li Ying, Shijiazhuang, Hebei Province (CN); CHENG, Xiao Xun, Shijiazhuang, Hebei Province (CN); MCHARDY, Nicholas, Blessington, Co. Wicklow (IE)
(74) Representative: Purdylucey Intellectual Property
(86) International application number: PCT/EP2021/074864
(87) International publication number: WO 2022/053584

(56) References cited:
- WO-A1-2010/127081
- WO-A1-2016/071446
- CN-A- 107 334 731
- GB-A- 2 185 395
- US-A- 3 795 737

## Description

### Introduction

This invention relates to a veterinary injectable formulation.

Buparvaquone has known activity against *Theileria* parasites. Imidocarb dipropionate has known activity against *Anaplasma, Babesia* ("large" forms only) and *Ehrlichia.*

WO2016/071446A describes an injectable formulation comprising buparvaquone and imidocarb dipropionate and contain water in an amount of 2%-15%.

CN107334731A discloses veterinary injectable composition comprising imidocarb and water and propanediol 40% (v/v) as a solvent.

The invention is directed to providing an improved formulation.

### Statements of Invention

The present invention is defined in the appended claims.

The references to methods of treatment in the statement of the invention and in the detailed description part in this description are to be interpreted as references to the compounds, pharmaceutical compositions and medicaments of the present invention for use in a method for treatment of the human (or animal) body by therapy.

According to the invention there is provided a veterinary single intramuscular injectable formulation comprising buparvaquone and imidocarb and salts thereof wherein the formulation is free from water.

The invention also provides a veterinary single intramuscular injectable formulation comprising buparvaquone and imidocarb or salts thereof, which is free from water, and a stabilising agent.

In one embodiment the stabilising agent is propyl glycol which provides a highly stable solution. Propyl glycol is 1,2-propylene glycol and is also known as propanediol.

In one case propyl glycol is in an amount of from 5% to 30% by weight of the composition. Propyl glycol may be in an amount of from 10% to 25% by weight of the composition.

In one case propyl glycol is in an amount of about 20% by weight of the composition.

The composition may comprise buparvaquone and imidocarb dipropionate. Buparvaquone may be present in an amount of about 50mg/ml. Imidocarb dipropionate may be present in an amount of about 48mg/ml.

In one embodiment the composition comprises N-methyl pyrrolidone.

In one case N-methyl pyrollidone is in an amount of from 20% to 70% by weight of the composition. N-methyl pyrollidone may be in an amount of from 40% to 60% by weight of the composition.

In one case N-methyl pyrollidone is in an amount of about 58% by weight of the composition.

In one embodiment the composition comprises a co-solvent. In one case the co-solvent is a fractionated coconut oil.

In one case the fractionated coconut oil is in an amount of from 3% to 30% by weight of the composition. The fractionated coconut oil may e in an amount of from 10% to 20% by weight of the composition.

In one case the fractionated coconut oil is in an amount of about 15% by weight of the composition. The composition may comprise a surface active agent.

Buparvaquone is completely insoluble in water. We have found that addition of water to organic solutions of buparvaquone causes the drug to be precipitated out of solution. Buparvaquone which precipitates remains at the injection site and has a much reduced therapeutic effect.

The formulation of the invention is in one case a co-solution of both buparvaquone and imidocarb dipropionate. Both active ingredients are completely in solution and therefore bioavailable.

A formulation that is a solution of the active ingredients is greatly preferable to any other injectable formulation type, such as a suspension or emulsion. Achievement of the formulation of the invention, therefore, is highly advantageous.

We have found that overtimes, even small amounts of water cause degeneration of imidocarb dipropionate, particularly in low concentrations in aqueous solutions.

We have found that imidocarb dipropionate does not dissolve in N-methyl pyrrolidone. However, we have surprisingly found that imidocarb dipropionate is soluble in a mixture of N-methyl pyrrolidone and propyl glycol. In addition, we have found that the solvents provide a formulation that is stable during various storage conditions. Not only is the formulation stable but also less toxic and causes less irritation as the amount of N-methyl pyrrolidone in the formulation can be reduced.

In the formulation of the invention, imidocarb dipropionate is mobilised slowly from the solution and over a relatively long period. Thus, relatively low but therapeutically effective blood concentrations are achieved over a relatively long period and short term anticholinergic effects are reduced.

The inclusion of fractionated coconut oil assists mobilisation of the active ingredients from the injection site and enhances the effectiveness of buparvaquone.

The invention also provides a method of treatment or prophylaxis of *Theileria* parasites and *Anaplasma, Babesia* and/or *Ehrlichia* parasites comprising administering an injectable formulation of the invention to a non-human animal. These parasites cause economically important diseases in cattle, sheep, goats, horses and dogs, particularly in tropical and sub-tropical countries.

*Theileria* and *Babesia* are protozoan members of the large and varied Class Sporozoa which also includes malaria and coccidial parasites. Sporozoans parasitise a wide range of animals, including mammals, reptiles, birds, fish and some invertebrates. The theilerias are believed to parasitise only herbivorous mammals but the host range of the babesias is far wider. Historically, the babesias were roughly divided into "large" and "small" forms but over the past 30 or so years some of the "small" forms have been re-classified as theilerias and other species. For example, the former *"Babesia equi"* is now recognised to be *Theileria equi.*

*Anaplasma* and *Ehrlichia* are primitive bacteria, totally unrelated to the Sporozoa.

Some hydroxynaphthoquinone compounds, including buparvaquone, are highly active against theilerias and some of the former "small" babesias, but they have no useful effect against the most widespread of the "large" babesias of cattle, including *B bigemina* and *B bovis.* However, they have some effect against others, including *B divergens,* which occurs mainly in northern Europe. Thus, no firm predictions can be made, without conducting individual studies, on whether a specific sporozoan parasite is susceptible to either buparvaquone or imidocarb, or to both compounds. It is very likely, however, that the present combination of buparvaquone and imidocarb would be effective against all diseases caused by theilerias and babesias, and at a minimum, most of those of uncertain classification that are closely related to these two groups.

Imidocarb dipropionate has no effect on the theilerias of cattle, but it is effective against *Th equi,* albeit at higher doses than against the large babesia of horses, *B caballi.* Imidocarb is unique in being effective against all the babesias of cattle and against bovine anaplasmosis, caused by *Anaplasma marginale,* and some ehrlichias, including *Ehrlichia canis.*

In one case the formulation is administered in an amount of about 1ml per 20kg bodyweight in cattle, sheep, goats, and horses.

In another case the formulation is administered in an amount of about 1ml per 15kg bodyweight in calves and dogs.

### Detailed Description

The invention will be more clearly understood from the following description of an embodiment thereof, given by way of example only.

The injectable formulation of the invention is a unique combination of the actives buparvaquone and imidocarb dipropionate. The two activities have a diverse solubility profile.

### Example 1 - Comparative

### List of Actives and excipients

1) Buparvaquone 50mg per ml.
2) Imidocarb 48mg per ml.
3) N-Methyl Pyrrolidone (NMP) Range 20% to 70% by weight.
4) Miglyol 812 (Fractionated coconut oil) and series of Miglyol e.g. 814, 840, 818. Conc. Range 3% to 36% by weight.
5) Water for Injection. Up to 10% by weight.
6) Any other surface active agent e.g. Sorbitan Monooleate (Span 80) conc. Range 0-5% by weight.

NMP is used as a solvent for buparvaquone. Miglyols are used as a co-solvent and to control the rate of depletion/absorption of buparvaquone and also to reduce irritation due to Imidocarb dipropionate at the injection site. Water for injection is used as a solvent for Imidocarb dipropionate. Surface active agents, such as Sorbitan, Spans and the like may be used as a co-solvent and as an emulsifier.

### Example 2

### List of Actives and excipients per ml

1) Buparvaquone 50mg.
2) Imidocarb dipropionate 48mg.
3) N-Methyl Pyrrolidone 600mg (75ml)
4) Propyl Glycol (PG) 200mg (20ml)
5) Miglyol 812 (Fractionated coconut oil) and series of Miglyol e.g. 814, 840, 818. Conc. Range 150mg (15ml)

### Preparation Method

1) Add PG to NMP and mix.
2) Add Imidocarb dipropionate to the mixed solvent, mix to almost dissolve.
3) Add buparvaquone and mix.
4) Add Miglyol and mix until clear.
5) Adjust volume with NMP, if necessary.

### Example 3 - Stability Testing

The formulation of Example 1 is a composition described in WO2016/071446A. The formulation of Example 2 is a composition according to the invention which was prepared as described above. The formulation of Example 2 has greatly enhanced stability when compared to the formulation of Example 1.

**Table 1:**

| | **Formulation 1** | **Formulation 2** |
|---|---|---|
| **BPQ** | 5.0g | 5.0g |
| **IMDP** | 4.8g | 4.8g |
| **NMP** | 75ml | 58ml |
| **Miglyol 812** | 15ml | 15ml |
| **Propanediol** | N/A | 20ml |
| **Water** | 3ml | N/A |
| **Total** | 100ml | 100ml |

**Table 2:**

| **Comparison of stability test result - ACC 40°C / RH75%** | | | |
|---|---|---|---|
| | | **Formulation 1** | **Formulation 2** |
| **Original** | IMDP | 102.23 | 99.20 |
| | BPQ | 96.92 | 95.99 |
| **1 month** | IMDP | 90.63/-11.6 | 97.08 |
| | BPQ | 89.29 | 95.73 |
| **2 month** | IMDP | 74.91 | 94.39 |
| | BPQ | 81.64 | 95.15 |
| **3 month** | IMDP | 68.15 | 93.62 |
| | BPQ | 75.90 | 92.45 |
| **6 month** | IMDP | 43.94 | |
| | BPQ | 59.01 | |

**Table 3:**

| **Comparison of stability test result - ACC 30°C / RH65%** | | | |
|---|---|---|---|
| | | **Formulation 1** | **Formulation 2** |
| **Original** | IMDP | 104.25 | 99.20 |
| | BPQ | 95.11 | 95.99 |
| **1 month** | IMDP | 100.58 | |
| | BPQ | 91.03 | |
| **2 month** | IMDP | 97.25 | |
| | BPQ | 88.78 | |
| **3 month** | IMDP | 93.8 | |
| | BPQ | 85.5 | |

**Table 4:**

| **Comparison of informal stability test result - LT/RT** | | | |
|---|---|---|---|
| | | **Formulation 1** | **Formulation 2** |
| **Original** | IMDP | 102.23 | 99.20 |
| | BPQ | 96.62 | 95.99 |
| 1 **month** | IMDP | 100.08 | |
| | BPQ | 94.31 | |
| **2 month** | IMDP | 94.93/99.08 | |
| | BPQ | 93.63/93.45 | |
| **3 month** | IMDP | 97.55/96.29 | |
| | BPQ | 93.51/93.23 | |
| **6 month** | IMDP | 91.18/91.50 | |
| | BPQ | 90.20/89.11 | |
| **12 month** | IMDP | 83.33/87.24 | |
| | BPQ | 85.81/88.35 | |

| | | | |
|---|---|---|---|
| RT - Room temperature, 10~30°C, LT-25°C 60%RH | | | |

**Table 5:**

| **Long term stability testing of the formulation of Example 2 - 25°C 60%RH** | | |
|---|---|---|
| | | **Formulation 2** |
| **Original** | IMDP | 105.79 |
| | BPQ | 102.29 |
| **3 month** | IMDP | 103.58 |
| | BPQ | 101.12 |
| **6 month** | IMDP | 102.69 |
| | BPQ | 100.59 |
| **9 month** | IMDP | 105.97 |
| | BPQ | 101.36 |
| **12 month** | IMDP | 104.1 |
| | BPQ | 100.39 |
| **18 month** | IMDP | 101.61 |
| | BPQ | 99.25 |
| **24 month** | IMDP | 100.89 |
| | BPQ | 98.053 |

### Therapeutic advantages - in cattle

Cases of theileriosis in cattle are frequently complicated by concurrent infection with *Anaplasma* and/or *Babesia.* All three are transmitted by ticks. The clinical signs of theileriosis may mask the signs of the other infections, which if untreated, are likely to kill the animal even if the theileriosis is cured. A single product that cures all three diseases has clear advantages.

Theileriosis causes severe immunedepression in its later stages. This can result in infections with *Anaplasma* and/or *Babesia* that were not clinically apparent at the time of treatment for theileriosis, becoming clinically significant after the theileriosis is cured. The veterinarian may assume that these signs indicate that the theileriosis was not cured by the initial treatment, so he gives additional treatment with the *Theileria* drug, which has no effect on either *Anaplasma* or *Babesia.* The correct treatment would be with imidocarb dipropionate. The animal, already weakened by theileriosis, is therefore very likely to die of anaplasmosis or babesiosis. Use of the formulation of the invention, which cures all three diseases, avoids this misdiagnosis problem. A combination product containing both active ingredients is convenient and less costly than individual doses, in terms of drug and of syringes and needles, and it saves time and causes less stress to the treated animal.

Cattle can be immunised against all forms of theileriosis (East Coast fever, Corridor Disease and tropical theileriosis) by a simple procedure of natural exposure to infection by ticks and treatment (NEAT) with the antitheilerial drug buparvaquone during the early stages of the subsequent disease. However, because anaplasmosis and babesiosis are also transmitted by ticks, there is a risk that these diseases will also be contracted. The use of a single product to treat all three diseases removes the risk that the additional diseases will go undiagnosed and therefore untreated, and it may also result in immunisation against anaplasmosis and babesiosis, as well as theileriosis.

### Therapeutic advantages - in horses

Horses in countries from France to South Africa and Australia, and China to the Americas are affected by two tick-transmitted diseases of the blood, caused by *Babesia caballi* (a "large" babesia) and *Theileria equi* which, until recently, was regarded as a "small" Babesia *- Babesia equi.* Imidocarb dipropionate was the drug of choice. It was very effective, in a single dose, against *Babesia caballi* but less so against *"Babesia equi",* which required up to four injections, each of them at double the dose for *Babesia* caballi. The formulation of the invention will usually cure both diseases with a single injection - the imidocarb dipropionate treating *Babesia caballi* infections and buparvaquone treating the *Theileria equi* (with some additional effect from the imidocarb dipropionate). A single product that treats both diseases has obvious advantages over two separate ones, and the specific antitheilerial effect of buparvaquone has inherent advantages over imidocarb dipropionate whose antitheilerial effects are relatively modest. Mixed infections with both parasites are common in some areas, but *Theileria equi* infection is likely to be overlooked when blood smears are used as an aid to diagnosis because the *Babesia caballi* parasites are far more conspicuous, and the clinical signs of the two diseases (particularly anaemia) are similar. Despite its smaller size, *Theileria equi* infection is more frequently fatal than *Babesia caballi* so a single product that cures both without the need for differential diagnosis is clearly advantageous.

While treatment of horses with imidocarb dipropionate readily eliminates *Babesia caballi* infection totally, it does not consistently eliminate *Theileria equi.* Treated horses may therefore remain asymptomatic carriers of this infection. When severely stressed, such "carriers" may again develop clinical disease. Carrier and clinically sick horses can be a source of infection for ticks, so an otherwise disease-free area can become infected, posing a big risk to all other horses.

Treatment with a single injection containing buparvaquone and imidocarb dipropionate will greatly increase the chance, and perhaps ensure, that *Theileria equi* infections will be completely eliminated by the treatment, thus minimising or removing the risk from treated "carrier" horses.

Clinical theileriosis or babesiosis will adversely affect the performance of horses. There is also some evidence that even the asymptomatic carrier state of these diseases may reduce the performance of race horses. If so, then a product that could completely eliminate the infections could be valuable, while not infringing anti-doping regulations.

Because it is such a severe disease and so difficult to cure, certain countries, most notably USA, prohibit the importation of horses from *Theileria equi*-endemic areas, including the Middle East and South America, unless it can be proven that they are not carriers of the infection. This involves quarantine for six months before importation, during which time the horses must be repeatedly "screened" to prove absence of the infection. This is clearly very expensive, particularly if, for example, an Arab stallion is to be imported. The formulations of the invention which reliably eliminates the infection, would be extremely valuable since proof that it had been used to treat the animal could be taken as proof that the horse was free of *Theileria equi* infection, obviating the need for quarantine.

### Therapeutic advantages - in dogs

Dogs in tropical and sub-tropical countries, and certain more temperate countries such as France, Italy, Spain and USA, are affected by at least three tick-transmitted blood diseases. *Babesia canis* (canine biliary fever) and most forms of *Ehrlichia (Anaplasma) canis* (including Nairobi bleeding disease) are readily treated by imidocarb dipropionate, albeit with relatively high treatment doses that may have to be repeated. However, several so-called "small babesias" are completely unaffected by this drug. These parasites however, are highly susceptible to treatment with buparvaquone. While these parasites are not really babesias, they are not theilerias either, because theilerias are exclusively parasites of herbivores, not carnivores. Currently, no veterinary drugs are available to treat them effectively.

Mixed infections of two or all three of these parasites are common, and the "small babesias" and ehrlichiosis can be difficult to detect, particularly in the presence of *Babesia canis,* so as with horse and cattle infections, a product that treats all three diseases without requiring differential diagnosis has clear advantages over imidocarb dipropionate alone.

### Formulation considerations

Buparvaquone at 50mg/ml, is regarded as the "ideal" formulation for the treatment of theileriosis in cattle. It reaches therapeutic concentrations within two hours of injection and its effect lasts for about three days. A single intramuscular injection cures most cases, although a second injection may be needed in more severe cases. The formulation of the invention is based on that of buparvaquone, so its therapeutic effect against theileriosis is predicted to be unchanged. Buparvaquone is completely insoluble in water. Addition of water to organic solutions of buparvaquone causes the drug to be precipitated out of solution. Buparvaquone which precipitates remains at the injection site, so it has very little therapeutic effect.

Imidocarb dipropionate, when used alone, is formulated as a 120mg/ml solution in water. This high concentration is used because lower concentrations are unstable over time. It reaches therapeutic concentrations in less than an hour after intramuscular injection and its effect against *Anaplasma* also lasts for about three days, but it is effective against babesias for much longer. Imidocarb, while highly soluble in water, is totally insoluble in organic solvents.

The formulation of the invention is in one case a co-solution of both buparvaquone and imidocarb dipropionate. Both active ingredients are completely in solution and therefore bio-available. A formulation that is a solution of the active ingredients is greatly preferable to any other injectable formulation type, such as a suspension or emulsion. Achievement of the formulation of the invention, therefore, is both novel and highly advantageous.

In the formulation of the invention both imidocarb dipropionate and buparvaquone are in solution in an overwhelmingly organic vehicle. This is surprising, particularly in view of the high concentrations of each of the active compounds in the formulation.

### Pharmacokinetic and toxicological considerations

Buparvaquone is almost uniquely non-toxic to treated animals and human operators. The absolute minimum toxic dose is at least 100-times the therapeutic dose. Imidocarb is significantly more toxic. Even at the normal therapeutic dose of 2.4 mg/kg bodyweight, it can cause notable (but reversible) adverse side effects and severe pain and swelling at the injection site in some (indeed most) cases, particularly in dogs and horses. This is almost certainly because it reaches very high (unnecessarily high for therapeutic purposes) concentrations in the blood very soon after injection. However, its concentration then falls to more "acceptable" levels in a few hours. The minimum lethal dose of imidocarb dipropionate is only around 9.6 mg/kg bodyweight (i.e. four times the therapeutic dose) when injected intramuscularly as an aqueous solution.

It is anticipated that in the formulation of the invention the imidocarb dipropionate will leave the injection site significantly more slowly, and over a much more protracted time-scale, than from a 120 mg/ml aqueous solution. This is expected to result in significantly less pain and swelling, and all other adverse side effects. This is a notable advantage over current imidocarb dipropionate products, particularly for horses and dogs, whose owners, understandably, dislike painful treatments.

While the time for the imidocarb dipropionate in the formulation of the invention to reach peak concentrations in the blood following injection will be delayed somewhat, its therapeutic effect and duration is unlikely to be reduced significantly. Thus, the therapeutic effect of the actives in the formulation against all its target diseases should be practically identical to that of the two active ingredients when injected as separate products.

The dose of the formulation of the invention will generally be between 1ml/10 Kg and 1ml/30Kg bodyweight, injected intramuscularly, in all animal species and for all target diseases. Additional similar injections may be necessary in more advanced cases of disease.

Generally the dose will be 1ml/20kg bodyweight in cattle and horses - i.e. the same as for buparvaquone, and up to 1ml/10 kg for some dog diseases. The dose volume for a 120 mg/ml aqueous formulation of imidocarb dipropionate, by contrast, is 1ml/50 or 100 kg, depending on the disease to be treated. This dose, while suitable for adult cattle and horses, is inconveniently small for calves, foals and dogs - particularly puppies, for which the dose may be less than 0.1 ml.

The invention is not limited to the embodiment hereinbefore described, which may be varied in detail.

## Claims

1. A veterinary single intramuscular injectable formulation comprising buparvaquone and imidocarb and salts thereof wherein the formulation is free from water.

2. A formulation as claimed in claim 1 wherein the formulation comprises a stabilising agent.

3. A formulation as claimed in claim 2 wherein the stabilising agent comprises propyl glycol.

4. A formulation as claimed in claim 3 wherein propyl glycol is in an amount of from 5% to 30% by weight of the composition.

5. A formulation as claimed in any of claims 1 to 4 comprising buparvaquone and imidocarb dipropionate.

6. A formulation as claimed in any of claims 1 to 5 wherein buparvaquone is in an amount of about 50mg/ml.

7. A formulation as claimed in claim 5 or 6 wherein imidocarb dipropionate is in an amount of about 48mg/ml.

8. A formulation as claimed in any of claims 1 to 7 comprising N-methyl pyrrolidone.

9. A formulation as claimed in claim 8 wherein N-methyl pyrrolidone is in an amount of from 20% to 70% by weight of the composition.

10. A formulation as claimed in any of claims 1 to 9 comprising a fractionated coconut oil.

11. A formulation as claimed in claim 10 wherein the fractionated coconut oil is in an amount of from 3% to 30% by weight of the composition.

12. A formulation as claimed in any of claims 1 to 11 comprising a surface active agent.

13. A veterinary single intramuscular injectable formulation for use in a method of treatment or prophylaxis of *Theileria* parasites and *Anaplasma, Babesia* and/or *Ehrlichia* parasites in a non-human animal wherein the formulation comprises buparvaquone and imidocarb and salts thereof and is free from water..

14. The veterinary single intramuscular injectable formulation for use in the method of Claim 13 wherein the formulation is administered in an amount of about 1ml per 20kg bodyweight in cattle, sheep, goats, and horses.

15. The veterinary single intramuscular injectable formulation for use in the method of Claim 13 wherein the formulation is administered in an amount of about 1ml per 15kg bodyweight in dogs.

## Patentansprüche

1. Veterinärmedizinische einmalige intramuskuläre injizierbare Formulierung, die Buparvaquon und Imidocarb und Salze davon umfasst, wobei die Formulierung wasserfrei ist.

2. Formulierung nach Anspruch 1, wobei die Formulierung ein Stabilisierungsmittel umfasst.

3. Formulierung nach Anspruch 2, wobei das Stabilisierungsmittel Propylglycol umfasst.

4. Formulierung nach Anspruch 3, wobei Propylglycol in einer Menge von 5 Gewichts-% bis 30 Gewichts-% der Zusammensetzung vorliegt.

5. Formulierung nach einem der Ansprüche 1 bis 4, die Buparvaquon und Imidocarb-Dipropionat umfasst.

6. Formulierung nach einem der Ansprüche 1 bis 5, wobei Buparvaquon in einer Menge von etwa 50 mg/ml vorliegt.

7. Formulierung nach Anspruch 5 oder 6, wobei Imidocarb-Dipropionat in einer Menge von etwa 48 mg/ml vorliegt.

8. Formulierung nach einem der Ansprüche 1 bis 7, die N-Methylpyrrolidon umfasst.

9. Formulierung nach Anspruch 8, wobei N-Methylpyrrolidon in einer Menge von 20 Gewichts-% bis 70 Gewichts-% der Zusammensetzung vorliegt.

10. Formulierung nach einem der Ansprüche 1 bis 9, die ein fraktioniertes Kokosnussöl umfasst.

11. Formulierung nach Anspruch 10, wobei das fraktionierte Kokosnussöl in einer Menge von 3 Gewichts-% bis 30 Gewichts-% der Zusammensetzung vorliegt.

12. Formulierung nach einem der Ansprüche 1 bis 11, die ein oberflächenaktives Mittel umfasst.

13. Veterinärmedizinische einmalige intramuskuläre injizierbare Formulierung für die Verwendung in einem Verfahren zur Behandlung oder Prophylaxe von *Theileria-Parasiten* und *Anaplasma-, Babesia-* und/oder *Ehrlichia-Parasiten* bei einem nicht-menschlichen Tier, wobei die Formulierung Buparvaquon und Imidocarb und Salze davon umfasst und wasserfrei ist.

14. Veterinärmedizinische einmalige intramuskuläre injizierbare Formulierung für die Verwendung in dem Verfahren nach Anspruch 13, wobei die Formulierung in einer Menge von etwa 1 ml pro 20 kg Körpergewicht bei Rindern, Schafen, Ziegen und Pferden verabreicht wird.

15. Veterinärmedizinische einmalige intramuskuläre injizierbare Formulierung für die Verwendung in dem Verfahren nach Anspruch 13, wobei die Formulierung in einer Menge von etwa 1 ml pro 15 kg Körpergewicht bei Hunden verabreicht wird.

## Revendications

1. Formulation vétérinaire unique injectable par voie intramusculaire comprenant de la buparvaquone et de l'imidocarbe et des sels de ceux-ci, la formulation étant exempte d'eau.

2. Formulation selon la revendication 1, la formulation comprenant un agent stabilisant.

3. Formulation selon la revendication 2, dans laquelle l'agent stabilisant comprend du propylène glycol.

4. Formulation selon la revendication 3, dans laquelle le propylène glycol est présent selon une quantité allant de 5% à 30% en poids de la composition.

5. Formulation selon l'une quelconque des revendications 1 à 4, comprenant de la buparvaquone et du dipropionate d'imidocarbe.

6. Formulation selon l'une quelconque des revendications 1 à 5, dans laquelle la buparvaquone est présente selon une quantité d'environ 50 mg/ml.

7. Formulation selon la revendication 5 ou 6, dans laquelle le dipropionate d'imidocarbe est présent selon une quantité d'environ 48 mg/ml.

8. Formulation selon l'une quelconque des revendications 1 à 7, comprenant de la N-méthylpyrrolidone.

9. Formulation selon la revendication 8, dans laquelle la N-méthylpyrrolidone est présente selon une quantité allant de 20% à 70% en poids de la composition.

10. Formulation selon l'une quelconque des revendications 1 à 9, comprenant une huile de noix de coco fractionnée.

11. Formulation selon la revendication 10, dans laquelle l'huile de coco fractionnée est présente selon une quantité allant de 3% à 30% en poids de la composition.

12. Formulation selon l'une quelconque des revendications 1 à 11, comprenant un agent tensioactif.

13. Formulation vétérinaire unique injectable par voie intramusculaire destinée à une utilisation dans une méthode de traitement ou de prophylaxie des parasites *Theileria* et des parasites *Anaplasma, Babesia* et/ou *Ehrlichia* chez un animal non humain, la formulation comprenant de la buparvaquone et de l'imidocarbe et des sels de ceux-ci, et étant exempte d'eau.

14. Formulation vétérinaire unique injectable par voie intramusculaire destinée à une utilisation dans la méthode selon la revendication 13, la formulation étant administrée selon une quantité d'environ 1 ml pour 20 kg de poids corporel chez les bovins, les moutons, les chèvres et les chevaux.

15. Formulation vétérinaire unique injectable par voie intramusculaire destinée à une utilisation dans la méthode selon la revendication 13, la formulation étant administrée selon une quantité d'environ 1 ml pour 15 kg de poids corporel chez les chiens.
